# EUROPEAN PATENT SPECIFICATION

(11) **EP 2 131 862 B1**
(45) Date of publication and mention of the grant of the patent: **06.10.2010**
(21) Application number: 08715610.5
(22) Date of filing: 06.03.2008
(51) Int. Cl.: A61K 41/00, A61P 17/00, A61P 17/10, A61K 31/195, A61K 8/44, A61Q 19/08, A61Q 9/00

(54) **Fuid photosensitizer for therapeutic photodynamic skin treatment and its use in non therapeutic photodynamic skin treatments**
Flüssiger Photosensibilisator zur therapeutischen photodynamischen Hautbehandlung sowie dessen Verwendung in nicht-therapeutischen photodynamischen Hautbehandlungsverfahren
Photosensibilisateur liquide pour le traitement photodynamique thérapeutique de la peau et son utilisation dans des procédés de traitement non thérapeutique de la peau

(30) Priority: 06.03.2007 WO PCT/DK2007/050029
(43) Date of publication of application: 16.12.2009
(73) Proprietor: Christiansen, Kåre, 2820 Gentofte (DK); Bjerring, Peter, 8240 Risskov (DK); Van Der Beek, Klaas, 3871KG Hoevelaken (NL)
(72) Inventor: Christiansen, Kåre, 2820 Gentofte (DK); Bjerring, Peter, 8240 Risskov (DK); Van Der Beek, Klaas, 3871KG Hoevelaken (NL)
(74) Representative: Rasmussen, Torben Ravn
(86) International application number: PCT/DK2008/050056
(87) International publication number: WO 2008/106983

(56) References cited:
- WO-A-03/086460
- WO-A-2005/092838
- US-A1- 2002 099 094
- US-A1- 2003 125 388

## Description

### Technological field of the invention

The invention relates to a method for non-therapeutic and a product for use in the therapeutic treatment of a skin target area of the mammal skin by a photodynamic process, by which a photosensitizer fluid incorporating a photosensitizing agent or a precursor for a photosensitizing agent, the photosensitizing agent having at least one absorption wavelength in a wavelength range higher than 400 nm, is delivered to the skin target area to produce in the skin tissue in the target area a distribution of a photosensitizing agent and the target area is exposed to light energy comprising a range of wavelengths including the at least one absorption wavelength of the photosensitizing agent distributed in the target area, the light energy being supplied to the target area in the form at least one pulse producing in the target area an accumulated energy density sufficient for photodynamic activation of the photosensitizing agent.

Non-therapeutic skin treatment, to which the invention pertains, includes in particular, but not exclusively treatment of photo damaged skin like pigmented disorders, treatment of telangiectasias and erythema as well as skin rejuvenation of dermal structures such as wrinkles, fine lines and skin pore size, and removal of unwanted hair.

Equally the invention is potentially useful to the medical treatment of dermatologic diseases and disorders including in particular, but not exclusively conditions such as actinic keratosis, basal, squamos and intraepithelial cell carcinoma, Bowen's disease, acne and seborrhoea, psoriasis and eczema.

### Background of the invention

Since the late 1980'es treatment of dermatologic conditions based on photodynamic activation of photosensitizing agents in affected skin regions has been proposed and reported by many scientists and disclosed in a number of patents.

Generally the proposed methods have suggested the administration of 5-aminolevulinic acid (5-ALA) or other precursors for production of the photosensitizing agent protoporphyrin IX to the affected skin region. After uptake in skin tissue 5-ALA and other precursors having similar effect metabolize in the epidermal cells to produce protoporphyrin IX, which absorbs light in the visible region of the spectrum of electromagnetic radiation and by exposure to such radiation reacts with oxygen in the cells to cause generation of free singlet oxygen radicals leading to destruction of the cell membrane and thereby cell necrosis.

US-A-5,079,262 (Kennedy et al) discloses treatment of an affected skin region by topical or oral application or injection of a relatively high concentration of 17 to 33% of ALA in a Glaxal base followed by exposure to photo activating light generally three hours after exposure.

In US-B2-6,897,238 (Anderson) the topical application of 5-ALA with a relatively low concentration of 0.1 to 1.0 % is disclosed as being effective for the treatment of acne vulgaris, acne rosacea and sebaceous gland hyperplasia by destruction of the bacteria associated with acne with the reservation, however, that the low accumulation of photosensitizer obtained hereby may require relatively frequent repetition of the treatment and proposing a relatively high concentration of 10 to 30 % 5-ALA for a suitable permanent treatment. As a main procedure for application the formulation of 5-ALA in a cream or emulsion, that can penetrate the skin, is disclosed, combined as a general practice with covering of the treated area with an occlusion material such as plastic for a period of 1 to 12 hours before activation of the photosensitizer by exposure to light energy. In a specific embodiment the use of a liposome as carrier by encapsulation of a low 5-ALA concentration combined with active driving of the carrier into follicles by massage, pressure, ultrasound or iontophoresis is disclosed.

In WO 03/086460 (Geronemus et al) the activation of a photosensitizer in an affected skin region by pulsed exposure to light energy with an elevated fluence rate, primarily from a pulsed dye laser has been disclosed. The main method proposed therein for 5-ALA administration is again the topical application of 20% 5-ALA formulated into a cream or emulsion to an affected skin area followed by covering the affected area with an occlusive dressing during a period of 10 minutes to 18 hours for incubation in a low light environment prior to the exposure to irradiating light energy.

Although focussing mainly on the medical treatment of essential malign or severe dermatological conditions the use of the described photodynamic treatment procedure has been proposed, e.g. in WO 03/086460, also as a recommendable vehicle for the cosmetic treatment of photoaged facial skin and the removal of unwanted, in particular, blonde, grey or light-coloured hair, this use involving the administration of 5-ALA as component of an alcohol solution with a typical concentration of 20 %.

In clinical practice it has turned out, however, that the beneficial effects of the photodynamic treatment are accompanied by side effects, in particular in the form of a relative long-lasting post-treatment period of heavy phototoxicity, typically for 24 to 48 hours, for the duration of which patients must be warned against skin exposure to bright daylight.

Another disadvantage of the recommended prior art procedures has been the relatively long duration of the treatment, in particular following from the need for covering the skin area under treatment with an occlusive dressing for a prolonged period of up till 18 hours for incubation of the 5-ALA into the active photosensitizing agent protoporphyrin IX.

Whereas such inconveniences may seen as although uncomfortable, yet acceptable conditions in the medical treatment of significant dermatologic diseases or disorders, they would be far from desirable in the cosmetic treatment of frequently occurring more daily life adverse skin conditions typically calling for treatment in the form of skin rejuvenation often performed by clinics outside a hospital environment.

### Summary of the invention

On the background outlined above, it is the overall object of the present invention to provide a novel photodynamic treatment procedure suitable for non-therapeutic applications or mainly cosmetic dermatologic treatment as well as a product for use in the medical treatment of dermatologic diseases and disorders, by which the described inconveniences of prior art procedures are significantly reduced.

It is a further object of the invention to provide a photodynamic treatment method which, while offering patients beneficial treatment results compared to the best state of the art procedures, can be easily practiced in non-therapeutical applications within a generally shortened overall duration of the procedure and with higher certainty against harmful and undesirable effects to patients by offering improved options for monitoring the progress of the treatment.

To accomplish these and other objects, which will become clear from the following description, the invention provides a method for non-therapeutic an a product for use in the therapeutic photodynamic treatment of a skin target area, comprising the steps of delivery of a photosensitizer fluid incorporating a photosensitizing agent or a precursor for a photosensitizing agent, the photosensitizing agent having at least one absorption wavelength in a wavelength range higher than 400 nm, to the target area, and exposure of the target area to light energy in a range of wavelengths comprising said at least one absorption wavelength of the photosensitizing agent, the light energy being supplied to the target area in the form of at least one light pulse producing in the target area an accumulated energy density sufficient for photodynamic activation of the photosensitizing agent.

According to the invention in its general aspect this method is **characterized in that** the photosensitizing agent or precursor is incorporated in the photosensitizer fluid with a concentration in the range from 0.1 to 2.0 % and that the photosensitizer fluid is delivered to the target area in the form of a number of repeated spray doses during an overall delivery time ranging from 0.25 to 8 hours.

The present invention builds on the realisation that multiple spray dosages of a photosensitizing fluid containing a relatively low concentration of the active principle leads to the accumulation of active principle in the skin in a controlled manner. Surprisingly, the repeated dosing of the photosensitizer fluid resulted in a phototoxicity rapidly decaying after the treatment.

By the term "a photosensitizer fluid" as used herein should be understood any pharmaceutically acceptable carrier fluid capable of being sprayed and carrying a photosensitizing agent or a precursor into a desired treatment region of the epidermis by penetration through outer layers of the skin.

The active component for production of the desired level of photosensitivity in the treatment region could be any photosensitizing agent, which by exposure to light is capable of causing injury to skin tissue and/or increasing fibroblast production in the skin and/or increasing production of collagen or elastic fibres. The exact explanation for the effect observed is at present not known.

As known in the art, there are many potential candidates for use as a photosensitizing agent for direct incorporation in the photosensitizer fluid of the invention. Preferably, the photosensitizing agent would comprise at least one of chlorine, a cyanine, a purpurin, a porphyrin and xanthenes.

From this group the more preferred photosensitizing agent would currently comprise a porphyrin and more preferably benzoporphyrin derivative monoacid.

As in most of the described prior art applications, the photosensitizer fluid would preferably include, however, a precursor for a photosensitizing agent capable of producing a distribution of the photosensitizing agent in the skin tissue in the target area by metabolic transformation.

In the currently most preferred implementation of the method of the invention, the precursor comprises 5-aminolevulinic acid (5-ALA), which when entering a target treatment skin region will cause production of the photosensitizing agent protoporphyrin IX.

Alternatively, the precursor may be an ALA ester selected among ALA-methyl ester, ALA-n-pentyl ester, ALA-n-octyl ester, R, S-ALA-2-(hydroxymethyl)tetrahydro-pyranyl ester, N-acetyl-ALA or N-acetyl-ALA-ethyl ester.

The explanation why a pre-treatment with this relatively low concentration 5-ALA leading to increased fibroblast production in the dermis is for the time being not known. According to one theory the photosensitizing agent generates when exposed to light free oxygen radicals leading to injury to the skin tissue. Thus, the effect might be of photodynamic nature. Photodynamic has been known since 1904 and was first described by Von Tappeiener and Jodblauer. In 1990 Kennedy et al first introduced 5-ALA as a topical photosensitizing agent, a pro-drug which could be transformed in the cells into the highly photoactive endogenous protoporphyrin IX (PpIX). The PpIX is activated by illuminating the skin surface with light of specific visible wavelengths (blue, yellow, red), which cause formation of free oxygen radicals leading in turn to cell necrosis. With 5-ALA in the relatively low dose of below 2%, few scattered cell destruction could result in that the biological signal of tissue reconstruction is needed leading to increased fibroblast production. According to another theory, 5-ALA is used as a precursor to several cytochroms. Supplying 5-ALA to the skin may increase the numbers of cytochroms and thereby the metabolism of the cells, leading to an increased production of collagen and elastic fibers.

Whereas the photosensitizer carrier fluid for use in the method of the invention would as already mentioned comprise any pharmaceutically acceptable carrier fluid capable of carrying a photosensitizing agent or a precursor in a desired treatment region of the epidermis by penetration through outer layers of the skin, a currently particularly preferred photosensitizer fluid would comprise a liposome preparation incorporating the photosensitizing agent or precursor by encapsulation in liposome vesicles. As known in the art typical liposome preparations are available, which offer excellent characteristics in terms of capability of penetration through outer dermal layers such as the stratum corneum.

A currently particularly preferred photosensitizer carrier fluid comprises a liposome preparation prepared from a nanokolloid liposome emulsion having a mean vesicle diameter less than 250 nm.

Liposome preparations having this characteristic have been disclosed in EP-A-1 013 265 and are available e.g. in the form of a phosphatidylcholine based concentrate available from DIANORM G. Maierhofer GmbH, München, DE. It is typically a low viscosity fluid, which is excellently suitable for application to the skin by conventional dispensing methods including spraying and can thereby be made available to biological processes in subsurface skin regions. The small liposomes have the ability easier to penetrate the skin surface (stratum corneum) and reach the cells below.

Whereas, with respect to prior art treatment procedures as described above it has been demonstrated that the prolonged post-treatment phototoxicity is mainly caused by a continued increase in the production of the photoactive component protoporphyrin IX for a considerable period after termination of 5-ALA administration, typically 8 to 10 hours, until a maximum level is reached, it has been demonstrated that in use of the procedure according to invention with a regular time interval of 5 or 15 minutes between the repeated spray doses the concentration of the photoactive component will, depending of the location of the treated area, reach its maximum shortly after termination of the administration of the precursor typically15-60 minutes after delivery of the last spray dose and will return to its base level within about 8 to 10 hours. In general, the maximum is reached faster on the back than on the face.

In result, both of the inconveniences of the prior art procedures described above may be significantly reduced by performing the exposure of the target area with light energy immediately after delivery of the photosensitizer fluid by a number of repeated spray doses and the decay of the concentration of the photoactive component in the target area within a shorter time after the treatment. In particular, this would be a major benefit to the application of the treatment regime of the invention to non-therapeutic cosmetic skin treatment to meet demands for the shortest possible period of post-treatment photo-toxicity as well as the shortest possible overall duration of a treatment session.

The spray dosages may be delivered to the target area using various intervals between the repeated spray dosages. In a certain aspect of the invention the spray doses is delivered to the target area with intervals of 2 to 30 minutes. Dosage intervals below 2 minutes is generally not suitable under normal conditions because the subsequent spray will be performed before the carrier of the former dosage has evaporated. However, if the evaporation is forced using i.e. an optionally heated fan of air, application rates below 2 minutes can be obtained. Dosage intervals above 30 minutes are generally not used because the treatment time is prolonged without the benefit of in improved treatment.

Unexpectedly, the total treatment time can be reduced by reducing the spraying intervals. Thus, by delivering the spray dosages in an interval of between 3 and 10 minutes, preferably about 5 minutes, a considerable decrease in the time needed for the same effect can be obtained when comparing to treatment using 15 minutes spraying intervals. Experiments reported herein supports that a similar effect can be obtained by spraying at one hour using 5 minutes spraying intervals compared to two hours of spraying in 15 minutes intervals. Needless to say a shorter treatment time allows more patients to be treated using the same equipment.

For determination of essential parameters of the method according to the invention such as the number of spray doses and the length of intervals separating them as well as the duration and possible sequence of pulses and the totally delivered energy density by the exposure to light energy, it may be of significant advantage to monitor the progress of the treatment procedure by objective means such as repeated measuring of the skin surface fluorescence emitted from the target area in response to excitation of the photoactive component by exposure to light specifically adapted to such a fluorescence measurement.

The number of repeated spray dosages is determined by balancing the various parameters. Thus, the repeated spray dosages include at least 2 dosages, preferably at least 3 or more to obtain a sufficient accumulation of photosensitizing agent or a precursor thereof. Normally, the number of repeated spray dosages does not exceed 50 to avoid an extended treatment period. Under normal conditions the number of repeated spray doses is between 2 and 36, preferably between 3 and 36.

Usually, the number of repeated spray dosages is balanced with the delivering intervals. Thus, when the delivering intervals are increased the number of repeated spray dosages is decreased. In a certain embodiment, when the number of repeated spray dosages is between 2 and 12, then the spray dosages are delivered to the target area with intervals of 10 to 15 minutes. In another embodiment of the invention, when the number of repeated spray doses is between 3 and 36, then the spray doses are delivered to the target area with intervals of 3 to 10 minutes.

It may be desired to use 1 or more interruptions in the repeated spraying scheme, i.e. to have a time interval between two spray dosages which is longer than the normal intervals. In a certain embodiment the repeated spray dosages are interrupted by 1 to 3 major time intervals of 10 minutes to 2 hours. In another embodiment, the spray dosages are interrupted by a single major time interval of 20 minutes to 2 hours in the middle part of the treatment.

The light energy used according to the present invention is preferably of the pulsed light source. The wave length is chosen with due regard to the absorption wavelength of the photosensitizing agent. When the protoporphyrin IX is the active photosensitizing agent, it can be activated by light irradiation with visible light covering the Soret band (407 nm) or the Q-bands (505, 540, 580 and 635 nm). In a preferred aspect of the invention a light source emitting 400 - 720 nm is used because is covers the entire activation wavelengths of protoporphyrin IX. The needed light irradiation for an efficient treatment with a light source covering all the absorption wavelengths is generally in the range of 3 to 30 J/cm². Usually, the light irradiation amounts to approximately 10 J/cm².

The amount of photosensitizing fluid applied in each spray dosage is usually sufficient for wetting the skin with a confluent layer of fluid. After application of the photosensitizing fluid it may be rubbed carefully into the entire skin target area. When the face is treated, the photosensitizing fluid is rubbed into the entire face including upper and lower eyelids as well as around the lips.

Prior to the treatment of the patient's face according to the invention, the skin target area is suitably washed for about 1 minute with e.g. Ellipse Exfoliating gel or a similar product able to degrease the entire skin area and remove dead skin cells. The Exfoliating gel is removed with plenty of water and target skin area is dried.

A novel method and apparatus suitable for performance of fluorescence measurement during a dermatological treatment session has been disclosed in co-pending international patent application entitled "Method and apparatus for in vivo determination of skin surface fluorescence" in the name of Dia-Medico ApS, Denmark et al.

In the non-therapeutic applications of the method according to the invention, including procedures for skin rejuvenation, reduction of wrinkles, treatment of telangiectasia or removal of hair, the essential parameter for delivery of the photosensitizer fluid to the treatment area would preferably comprise incorporation of the photosensitizing agent in the photosensitizer fluid in a concentration from 0.1 to 2.0 %, preferably from 0.5 to 1.0 % and delivery to the target area in from 2 to 36 successive spray doses separated by intervals between 3 and 15 minutes.

Whereas application of the method according to the invention to medical treatment of dermatological conditions including acne vulgaris, acne scars, rosacea, psoriasis, actinic keratosis, basal cell carcinoma Bowen's disease or eczema has so far been conducted to a limited scale only, the indications currently available are that the same ranges of treatment parameters would be very useful also for therapeutic treatment.

In a second aspect, the invention further relates to a liposome based photosensitizer fluid for use in any of the described treatment procedures. Conforming to the currently preferred implementation of the method this photosensitizer fluid is characterized by comprising a nanokolloid liposomic emulsion having a mean vesicle diameter not exceeding 250 nm, in which a photosenzitizing agent or a precursor for a photosensitizing agent is encapsulated in liposome particles with a concentration in the range from 0.1 to 2.0 %.

In a still further aspect, the invention relates to the use of a photosensitizing agent or a precursor for a photosensitizing agent in the manufacture of a photosensitizer fluid for application in photodynamic treatment of a dermatologic disorder, wherein the photosensitizing agent or precursor is incorporated in the photosensitizer fluid with a concentration in the range from 0.1 to 2.0 % and the photosensitizer fluid is delivered to the target area in the form of a number of repeated spray doses during an overall delivery time ranging from 0.25 to 8 hours.

### Brief description of the drawings

In the following, the invention will be explained more in detail with reference to the accompanying drawings and by way of a clinical example.

In the appended drawings
figure 1 shows the application scheme for a fluorescence study with different concentration and carriers of 5_ALA
figures 2 to 4 are graphic representations of some of the results obtained by a preliminary investigation of treatment prospects of procedures according to invention compared to characteristics of established prior clinical practice for photodynamic therapy;
figure 5 is a graphic representation illustrating the potential benefit of the photodynamic procedure of the invention to medical therapy of actinic keratosis; and
figures 6 and 7 are graphic representations of results obtained in a clinical investigation of a photodynamic cosmetic treatment for reduction of wrinkles.
Figures 8 shows the averaged normalized fluorescence after 5 minutes spraying intervals performed on Caucasian skin type.
Figure 9 shows the averaged relative obtained fluorescence on Caucasian skin type by 5 minutes spraying intervals, compared to fluorescence obtained by 2 hours spraying with 15minutes intervals.
Figure 10 shows a diagram showing that the same fluorescence is obtained on Caucasian skin type with 5 minutes of spraying intervals as after 2 hours of spraying with 15 minutes intervals.
Figure 11 shows the result of an experiment using Asian skin types sprayed for 1 and 2 hours applying 5 minutes spraying intervals.

### Detailed description

As will appear from the foregoing description the mechanism of the use of 5-ALA in photodynamic therapy is based on intracellular transformation of 5-ALA into protoporphyrin IX followed by exposure to light energy, by which photo-toxicity is induced. The efficacy of the treatment is determined by a number of factors including a sufficient metabolic transformation rate for 5-ALA to obtain the intended phototoxicity upon subsequent light exposure, inclusion of the major absorption bands of protoporphyrin IX in the optical spectrum used for the light exposure and selection of the light dose in terms of energy density to be sufficient to obtain oxygen concentration in the treatment area.

Since in contrast to 5-ALA the photoactive component protoporphyrin IX produces a pronounced fluorescence at a wavelength of 634 nm by excitation with a wavelength of 407 nm, a preliminary investigation of the metabolic transformation of 5-ALA into protoporphyrin IX has been performed by detection of skin surface fluorescence, which provides a fair representation of the distribution and concentration of protoporphyrin IX in a skin region.

The investigation was conducted to obtain empirical verification of the assumption that use of liposome encapsulated 5-ALA in a low concentration would provide the benefits of the invention as compared to the conventional clinical practice of incorporation of a concentration of 20 % 5-ALA in a standard moisturizing cream and also to optimize treatment parameters like concentration and incubation time for 5-ALA to obtain a standardized fluorescence response from the protoporphyrin IX produced in the treatment region. The results of the investigation were obtained by skin fluorescence measurements implying visual assessment of fluorescence photographs obtained by a digital camera as well as objective fluorescence quantification by a novel fluorescence detection device as disclosed in the international patent application mentioned above.

This investigation was attended by ten healthy volunteers with no previous skin diseases and with a mean age of 25.7 years. In the administration of 5-ALA to skin target areas the photosensitizer carrier was a nanokolloid liposome preparation as described above and the investigation comprised preparations containing 1.0 and 0.5 % 5-ALA, respectively, which were applied to the skin in the form of repeated spray doses with regular intervals of 15 minutes. For comparison, the investigation included the administration of 5-ALA by way of a conventional standard moisturizing cream preparation containing 20 % 5-ALA, which was applied to the skin and followed by the standard procedure of covering the test area with an impermeable plastic occlusion film during the incubation period.

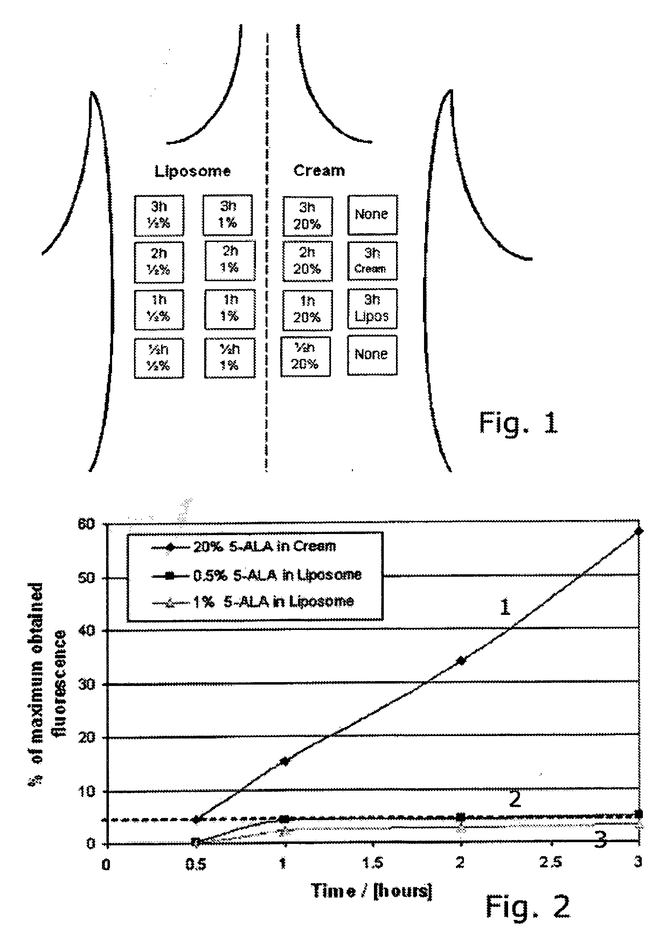

As schematically illustrated in figure 1, 16 test areas of equal size and in a 4 x 4 configuration were selected on the back of each of the participating volunteers. For each of the three defined 5-ALA preparations four test were used to investigate the effect of using incubation times of 30 minutes, 1 hour, 2 hours and 3 hours, respectively. The test areas in the remaining column in the 4 x 4 matrix was used for negative control and comprised two non-treated areas positioned adjacent the test area supplied with 20 % 5-ALA cream-based preparation for the shortest and longest incubation times of 30 minutes and 3 hours, respectively, whereas the two intermediate test areas were supplied with the moisturizing cream base and the liposome carrier emulsion both without any content of 5-ALA.

During the investigation, skin surface fluorescence was measured for all test areas before application of the 5-ALA preparations and the empty control preparations. For each test area subsequent fluorescence measurements were performed immediately after termination of the incubation time and every 30 minutes during the following 3.5 hours and ultimately with extended time intervals of 1 hour for the subsequent 7 hours.

The main results of the investigation are summarized in the graphic representations in figures 2, 3 and 4.

Figure 2 is a representation of the average increase for the ten volunteers of the fluorescence level measured at the end of the incubation time for each test area for each of the three 5-ALA preparations defined by the curve 1 for the cream-based 20 % 5-ALA concentration, the curve 2 for the 0.5 % 5-ALA liposome preparation and the curve 3 for 1.0 % 5-ALA liposome preparation. Whereas the curve 1 shows a continued linear increase in fluorescence level for the cream-based 20 % 5-ALA concentration with incubation time, the curves 2 and 3 show an approximate saturation in fluorescence level from an incubation time of 2 hours and a higher maximum fluorescence level for the lower 0.5 % 5-ALA concentration than for the 1 % concentration.

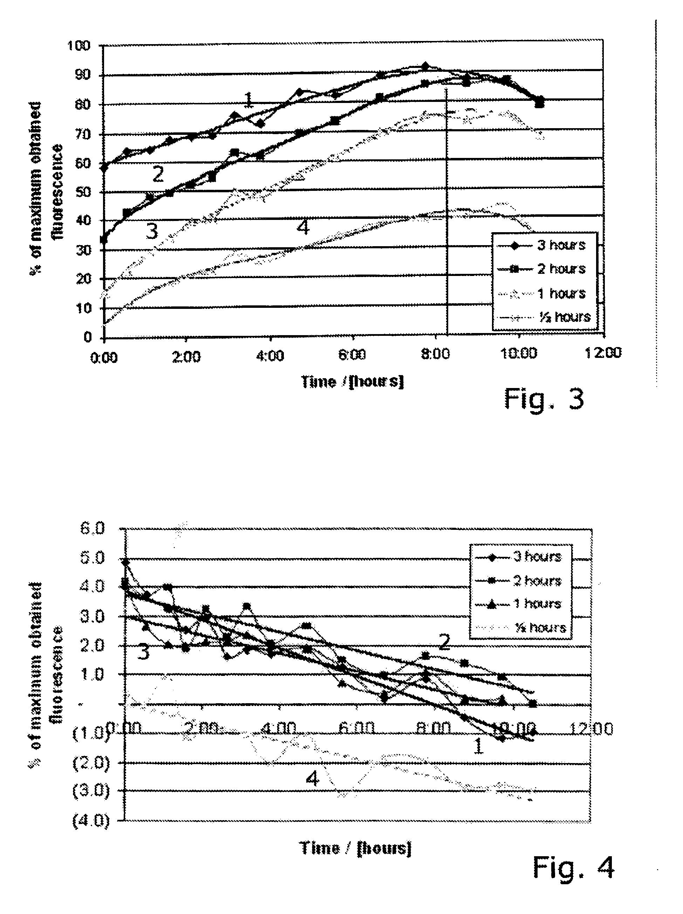

Figure 3 is a representation of the average increase for the ten volunteers of the fluorescence level measured during a period of 10 hours after the incubation period for the cream-based 20 % 5-ALA concentration administered to the skin with incubation times of 3 hours for curve 1, 2 hours for curve 2, 1 hours for curve 3 and 30 minutes for curve 4, respectively. The representation demonstrates that independent of incubation time the fluorescence level by administration of the cream-based 20 % 5-ALA concentration continues to increase to a saturation level, which is not reached until about 8 hours after termination of the incubation time. This provides a clear indication of the prolonged post-treatment period of phototoxicity, which is a main inconvenience in the use of this preparation for treatment purposes. Further the representation in figure 3 shows a statistically ignorable difference between the maximum fluorescence level obtained for the incubation times of 3 hours, 2 hours and 1 hour.

Figure 4 is a representation corresponding to figure 3, but showing an average decrease of the fluorescence level during the 10 hours period following termination of the incubation period for the 0.5 % 5-ALA liposome preparation administered to the skin with incubation times of 3 hours for curve 1, 2 hours for curve 2, 1 hour for curve 3 and 30 minutes for curve 4, respectively. The representation provides a clear indication that, by application of the method according to the invention with intervals of 15 minutes between deliveries of the repeated spray doses, the fluorescence level will independently of the duration of the incubation period start decaying within 15 minutes from termination of the incubation period, and the decay progresses at a rate, by which the fluorescence level is back to the base line after 8 to 10 hours. Like the representation in figure 3 also the representation in figure 4 illustrates a statistically ignorable difference between the maximum fluorescence levels obtained for the incubation times of 3 hours, 2 hours and 1 hour.

An additional visual observation made during the investigation was that, for eight out of the ten volunteers, the fluorescence level measured for application of the 0.5 % 5-ALA liposome preparation with incubation times of 1 hour and 2 hours, which are close to each other, as also confirmed by figure 2, were equivalent to the fluorescence level measured for application of the cream-based 20 % 5-ALA concentration with an incubation time of 30 minutes. As this relatively short incubation time for use of the conventional cream-based 5-ALA preparations have become clinical practice for cosmetic treatment in an attempt to reduce the prolonged period of post-treatment photo-toxicity, this observation provides a clear indication of the efficiency of the procedure according to the invention for use in non-therapeutic cosmetic skin treatment compared to the use of cream-based 5-ALA preparations.

An additional significant observation made during the investigation followed from the presence of localized acne inflammations in some test areas. Fluorescence measurement made in connection with administration of the 0.5 % 5-ALA liposome preparation to such test areas demonstrated a highly selective fluorescence concentrated in the inflamed acne lesions, whereas in skin areas treated with the cream-based 20 % 5-ALA preparation fluorescence was practically uniformly distributed over the detection area. This observation provides an indication of rapid transformation of 5-ALA into protoporphyrin IX in cells with a high metabolism and thereby a high potential utility for the application of the procedure according to the invention in the treatment of dermatologic diseases like acne vulgaris, acne scars, rosacea, psoriasis, actinic keratosis, basal cell carcinoma, Bowen's disease or eczema.

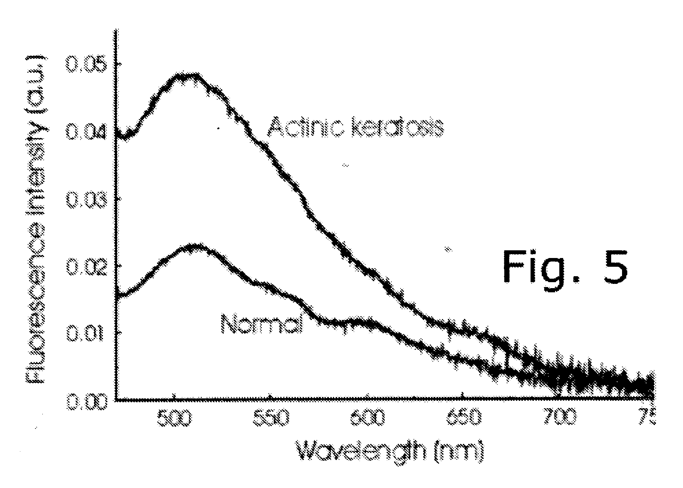

This observation is in line with other investigations made for administration of 5-ALA to the treatment of actinic keratosis, which as illustrated in the graphic representation in figure 5 also demonstrates a high selective fluorescence in cancer cells with high metabolism as compared to normal cells.

The usefulness of the method according to the invention to non-therapeutic skin rejuvenation procedures is confirmed by the following clinical examples:

### Example 1

A clinical multi-centre study of the effect of the method according to the invention to facial wrinkle reduction using 0.5% 5-ALA liposome preparation was attended to and finalized by 20 volunteers with ages ranging from 37 to 70 years and Fitzpatrick skin type varying from I to IV. Suntan in the treated areas was between none to heavy. The volunteers had visible periorbital and/or perioral wrinkles, on Fitzpatrick's wrinkle scale, periorbital wrinkles around the eyes averaged 5.88 and perioral wrinkles (around the mouth averaged 5.48. All patients were instructed to avoid sun exposure and use efficient sun protection every morning and prior to out door activities in sunny weather.

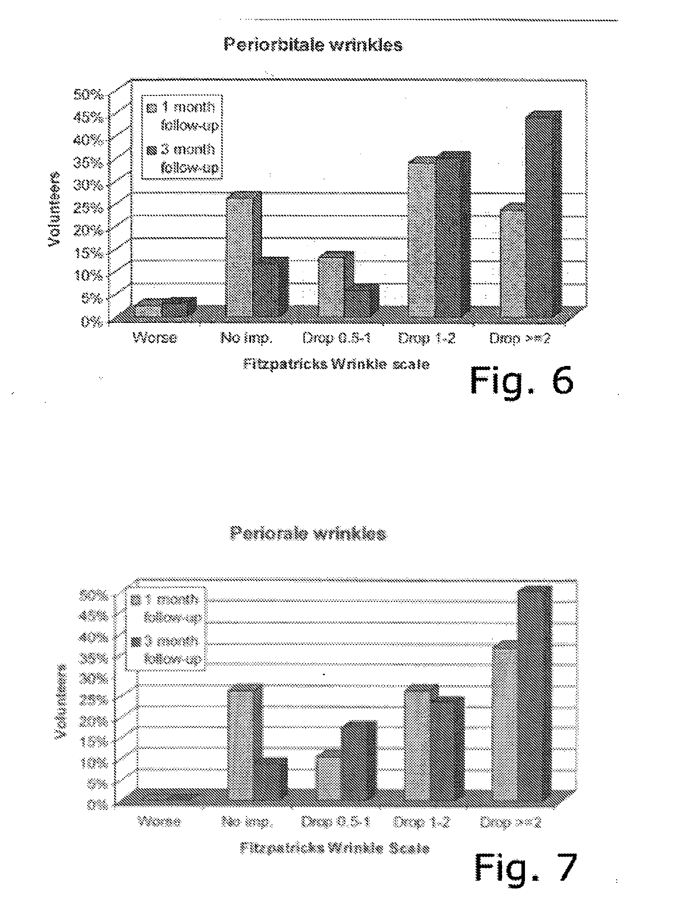

Wrinkle severity according to Fitzpatrick's wrinkle scale was evaluated before the start of the treatment and at 1 and 3 months post treatment follow-up sessions with the results for reduction in periorbital wrinkles and perioral wrinkles shown in the diagrams in figures 6 and 7, respectively.

The reduction in Fitzpatrick's wrinkle severity for periorbital wrinkles was in average 0.96 at the 1month follow-up session and increased to an average of 1.49 at the 3 months follow-up session. Corresponding figures for the reduction of perioral wrinkles were 1.2 at the 1 month follow-up and 1.57 at the 3 months follow-up sessions. The distribution with respect to perioral and periorbital wrinkles, respectively are shown in the representations in figures 6 and 7.

Wrinkle reduction and improvements in skin texture was evaluate at the 1 month follow-up session to be fair, good or excellent by 57.8 % and 43.8 %, respectively, of the patients. At the 3 months follow-up session these scores had increased to 64.5 % for wrinkle reduction as well as for skin texture.

The overall satisfaction of the patients with the treatment procedure was evaluated by the range: unsatisfied, slightly satisfied, satisfied, very satisfied and extremely satisfied. At the 1 and 3 months follow-up sessions 71.0 and 70.8, respectively, of the patients rated the treatment with a score satisfied or higher.

### Example 2

A clinical multi-centre study using 0.5% 5-ALA liposome preparation was finalized by a total number of 27 volunteers in the age range from 25 to 70 years and belonging to Fitzpatrick skin types I to III with a degree of suntan in the treated skin areas varying from none to heavy. The volunteers had visible periorbital and perioral wrinkles and/or fine lines and suffered from sun damages skin with benign vascular and pigmented lesions.

The volunteers finalizing the study were divided into three groups comprising 10, 8 and 9 volunteers, respectively, treated at individual clinical sites in Denmark, Sweden and The Netherlands.

The volunteers in group 1 received a full face spraying with a 0.5 % 5-ALA liposome spray delivered in 8 spray doses with intervals of 15 minutes for an overall duration of 2 hours immediately followed to exposure to light energy using the standard IPL (Intensified Pulse Light) equipment ELLIPSE® from Danish Dermatologic Development A/S. One side of the face was subjected to exposure with a standard applicator emitting a wavelength range from 530 to 750 nm and supplying an energy density of 7 J/cm² in a pulse train consisting of two pulses of a duration of 2.5 ms spaced by an interval of 10 ms. The opposite side was treated with the same IPL equipment using a special applicator emitting the wavelength range from 400 to 720 nm in three subsequent passes each with a single pulse of a duration of 30 ms and an energy density of 3.5 J/cm² adding up to a total delivered energy density of 10.5 J/cm².

The volunteers in group 2 had a randomly selected face side pre-sprayed in 12 doses with intervals of 15 minutes for an overall duration of 3 hours and the other face side pre-sprayed in 4 doses with intervals of 15 minutes for an overall duration of 1 hour, for both sides with the same liposome-based 5-ALA preparation as the group 1 patients. The exposure was conducted as a full face treatment using the same special applicator as for group 1 in three subsequent passes each with a single pulse of a duration of 30 ms and an energy density of 3.5 J/cm² adding up to a total delivered energy density of 10.5 J/cm².

The volunteers in group 3 had randomly selected face sides pre-sprayed for 3 hours and 1 hour, respectively, using the same spray delivery procedure as for group 2 and exposure was performed immediately after termination of the pre-spraying by use of the same equipment as for group 1 using the standard applicator emitting a wavelength range from 530 to 750 nm and supplying an energy density of 7 J/cm² in a pulse train consisting of two pulses of a duration of 2.5 ms spaced by an interval of 10 ms.

The conclusions of this study were
- Pre-spraying with the 0.5 % liposome encapsulated 5-ALA resulted in an improved reduction in wrinkles and fine lines compared to a standard photo rejuvenation procedure using light exposure without pre-spraying with the same standard applicator emitting a wavelength range from 530 to 750 nm and supplying an energy density of 7 J/cm² in a pulse train consisting of two pulses of a duration of 2.5 ms spaced by an interval of 10 ms: This improvement was observed without any statistically significant difference for the standard light applicator end the special applicator;
- For reduction of perioral wrinkles a statistically significant trend for higher efficacy for 3- hours pre-spraying over 1 hour pre-spraying was observed;
- For improvement in pigmentation, diffuse redness and telangiec-tasis a statistically significant superiority was observed for use of the standard light applicator emitting a wavelength range from 530 to 750 nm and supplying an energy density of 7 J/cm² in a pulse train consisting of two pulses of a rudation of 2.5 ms spaced by an interval of 10 ms;
- Superiority of the special light applicator emitting the wavelength range from 400 to 720 nm three subsequent passes each with a single pulse of a duration of 30 ms and an energy density of 3.5 J/cm² adding up to a total delivered energy density of 10.5 J/cm² was observed in the sense that it can be used for treatment of all skin types even medium to heavy pigmented ones up to skin type V on the Fitzpatrick scale.

### Example 3

The relative long lasting spraying period used in example 2 may be a disadvantage for a combined treatment modality. In this study we investigated the possibilities for shorten the spraying period by decreasing the intervals between spraying, and still obtain the same degree of fluorescence. The shortest practical possible time between two sprayings avoiding spraying wet in wet, is approximate 5 minutes. Therefore we have in this study chosen to compare obtained fluorescence after 5 min and 15 min spraying intervals. This study was performed as a split face study with random chosen spraying side for 15 min and 5 min which means the volunteers acts as their own control. 10 volunteers with Caucasian skin types were enrolled. Spraying was continued in two hours.

This study is based on fluorescence measurements performed under spraying normal facial skin with 0.5% liposome encapsulated 5-ALA, as well as in a period lasting up to 2 hours after end spraying. The test area has to be covered by a folio during the entire test period, avoiding direct or indirect light exposure to have influence on the fluorescence measurements.

Randomized site of face was chosen for 5 minutes spraying interval with liposome encapsulated 5-ALA. 15 minutes spraying intervals was performed on the opposite part of the face.

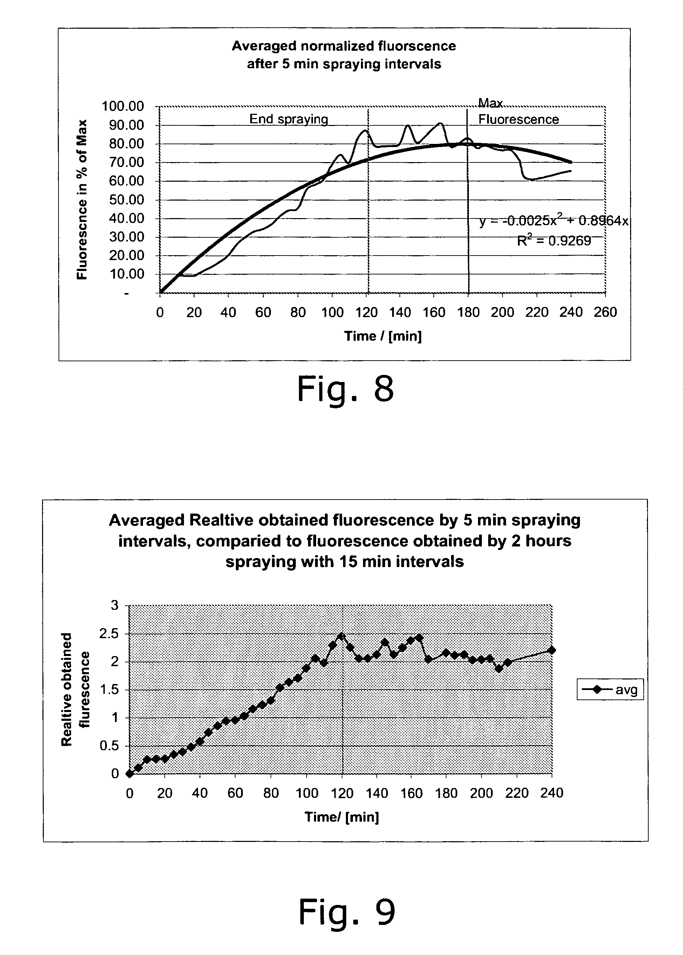

By taking the average of the normalized fluorescence data we find the curve in Fig. 8, which predicts that maximum fluorescence will occur 1 hour after 2 hour of spraying with 5 minutes intervals.

The difference between obtained fluorescence by 5 and 15 minutes spraying intervals was observed. In average, volunteers obtained 2.45 times higher fluorescence after 2 hours spraying with 5 minutes interval than with 2 hours spraying with 15 minutes interval as shown in Fig. 9. The difference was in according to a student-t paired test found to be statically significant for all teen volunteers measured as individuals. (0.048< p < 0.8*10-7).

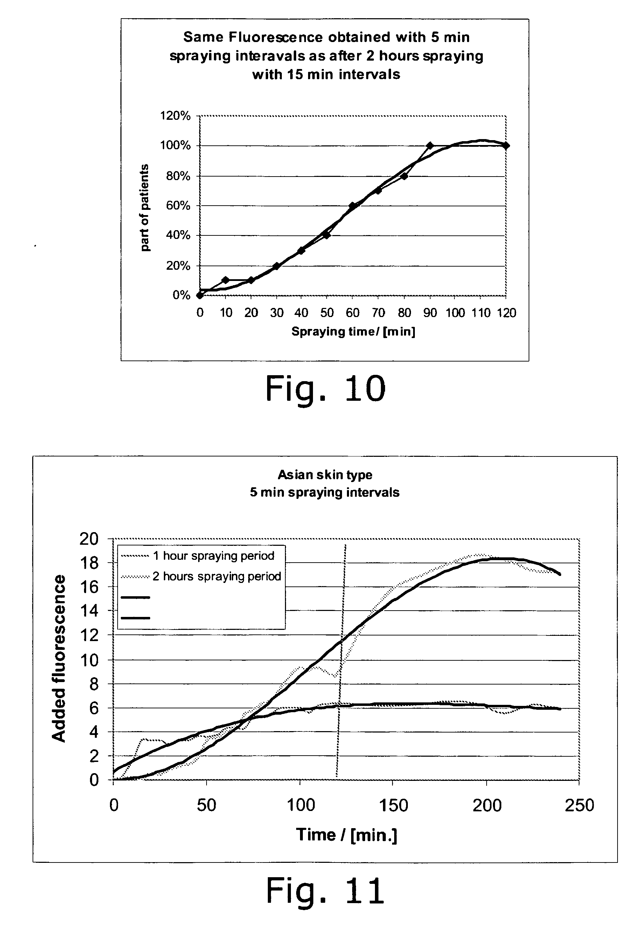

One the of the goals for this investigation was to determine the necessary numbers of spraying procedures with 5 minutes spraying intervals, which results in at least the same fluorescence level as obtained by the same patient after 2 hours spraying with 15 minutes intervals. From Fig. 10 it can be seen that all volunteers reach that level after approximate 90 minutes of spraying with 5 min intervals. However, from Fig. 9 it can be seen after 90 min spraying with 5 min intervals that an average in normalized fluorescence is obtained, which is 1.7 times higher than for 2 hours spraying with 15 min intervals.

If we look on obtained fluorescence after 1 hour of spraying with 5 min intervals then we can see 60 % of the volunteers have reached the level of 2 hours spraying with 15 min interval. This level is known to give nice reduction in wrinkles if an IPL treatment procedure is performed.

### Example 4

The test employed one hour of photo spray spraying with 5 min intervals followed by a pause up to 2 hours. 4 volunteers with Asian skin type enrolled in this investigation.

The face was pre-treated by first washing both cheeks 1 minute with Ellipse Exfoliating Gel. A randomized side of the face was left further untreated. The opposite side was additionally degreased by wiping 5 times wit a gaze swap moisturized with acetone and 5 times with a gaze swap moisturized with 93% alcohol.

Spraying with liposome Photo Spray with 5 minutes interval was performed on both cheeks for a period of 1 hour. Fluorescence measurements were performed with 5 minutes intervals during the spraying period as well as during 1 hour after end of Photo Spray application. In the remaining period fluorescence was measured every 15 minutes.

As shown in fig. 11 the averaged fluorescence reached its optimum 1 hour after 1 hour of spraying with Photo Spray at a level of 6.4 (SD.: 1.0) compared to 8,67 (SD.: 3,13) immediately after 2 hours of spraying with 5 min intervals. As the added fluorescence for all patients has reach the wanted minimum response level of 4 on the FluoDerm scale, already 15 minutes after end of 1 hour spraying with Photo Spray, and as the maximum level of the averaged added fluorescence (5.5 - 7.5) occurs 1 hour after end spraying, it is most likely that the outcome by a following IPL wrinkle treatment will be as efficient as after 2 hours spraying with 5 min intervals 8,67 (SD.:3,13).

Furthermore, from fig. 11 it can be seen that the span of time where the following IPL treatment can be performed with a stable added fluorescence is 100 - 200 minutes after 1 hour of spraying. This relatively short time span indicates that this treatment is a very safe treatment modality.

## Claims

1. A method for non-therapeutic photodynamic treatment of a skin target area, comprising the steps of
delivery of a photosensitizer fluid incorporating a photosensitizing agent or a precursor for a photosensitizing agent to the target area, the photosensitizing agent having at least one absorption wavelength in a wavelength range higher than 400 nm, and
exposure of the target area to light energy in a range of wavelengths comprising said at least one absorption wavelength of the photosensitizing agent, the light energy being supplied to the target area in the form of at least one light pulse producing in the target area an accumulated energy density sufficient for photodynamic activation of the photosensitizing agent,
**characterized in**
**that** the photosensitizing agent or precursor is incorporated in the photosensitizer fluid with a concentration in the range from 0.1 to 2.0 % and that the photosensitizer fluid is delivered to the target area in the form of a number of repeated spray doses during an overall delivery time ranging from 0.25 to 8 hours.

2. A method as claimed in claim 1, wherein the photosensitizer fluid incorporates a photosensitizing agent or a precursor for a photosensitizing agent capable of producing a distribution of the photosensitizing agent in the skin tissue in the target area by metabolic transformation.

3. A method as claimed in claim 2, wherein the precursor comprises aminolevulinic acid (ALA) or an ALA ester, selected among ALA-methyl ester, ALA-n-pentyl ester, ALA-n-octyl ester, R,S-ALA-2-(hydroxymethyl)tetrahydropyranyl ester, N-acetyl-ALA or N-acetyl-ALA-ethyl ester.

4. A method as claimed in any of claims 1 to 3, wherein the photosensitizer fluid comprises a liposome preparation incorporating the photosensitizing agent or precursor by encapsulation in liposome vesicles, said liposome preparation being prepared from a nanokolloid liposome emulsion having a mean vesicle diameter less than 250 nm.

5. A method as claimed in any of the claims 1 to 4, wherein the photosensitizing agent or precursor is incorporated in the photosensitizer fluid with a concentration in the range from 0.1 to 0.9%.

6. A method as claimed in any of the claims 1 to 5, wherein the number of repeated spray doses is between 2 and 36.

7. A method as claimed in any of the claims 1 to 6, wherein the spray doses is delivered to the target area with intervals of 2 to 30 minutes.

8. A method as claimed in any of claims 1 to 7, wherein the exposure of the target area to light energy is initiated with a time delay not exceeding 5 minutes from termination of said overall delivery time for the photosensitizer fluid to the target area.

9. A method as claimed in any of claims 1 to 8 for skin rejuvenation, reduction of wrinkles, treatment of telangiectasia or removal of hair.

10. A photosensitizing fluid incorporating a photosensitizing agent or a precursor thereof for use in a method for therapeutic photodynamic treatment of a skin target area, comprising the steps of
providing a photosensitizer fluid incorporating a photosensitizer agent having at least one absorption wavelength in a wavelength range higher than 400 nm or a precursor for such photosensitizing agent with a concentration in the range of from 0.1 to 2.0 %,
delivering the photosensitizer fluid to the target area,
exposing the target area to light energy in a range of wavelengths comprising said at least one absorption wavelength of the photosensitizing agent, the light energy being supplied to the target area in the form of at least one light pulse producing in the target area an accumulated energy density sufficient for photodynamic activation of the photosensitizing agent,
**characterized in that** the photosensitizer fluid is delivered to the target area in the form of a number of repeated spray doses during an overall delivery time ranging from 0.25 to 8 hours.

11. A photosensitizing fluid according to claim 10, for use in a treatment of acne vulgaris, acne scars, rosacea, psoriasis, actinic keratoses, basal cell carcinoma, Bowen's disease, malign dermatologic condition or eczema.

12. The photosensitizing fluid according to claims 10 or 11, wherein the photosensitizer fluid comprises a liposome preparation incorporating the photosensitizing agent or precursor by encapsulation in liposome vesicles having a mean vesicle diameter not exceeding 250 nm.

13. A photosensitizer fluid according to any of the claims 10 to 12, wherein the photosensitizing agent or precursor is incorporated in the photosensitizer fluid with a concentration in the range from 0.1 to 0.9%.

14. Use of a photosensitizer fluid incorporating a photosensitizer agent having at least one absorption wavelength in a wavelength range higher than 400 nm or a precursor for such photosensitizing agent, with a concentration in the range of from 0.1 to 2.0 % for non-therapeutic photodynamic treatment of a skin target area, wherein the photosensitizer fluid is delivered to the target area, the target area is exposed to light energy in a range of wavelengths comprising said at least one absorption wavelength of the photosensitizing agent, the light energy being supplied to the target area in the form of at least one light pulse producing in the target area an accumulated energy density sufficient for photodynamic activation of the photosensitizing agent, and the photosensitizer fluid is delivered to the target area in the form of a number of repeated spray doses during an overall delivery time ranging from 0.25 to 8 hours.

15. Use of a photosensitizing agent or a precursor for a photosensitizing agent in the manufacture of a photosensitizer fluid for application in photodynamic treatment by therapy of a skin condition, wherein the photosensitizing agent having at least one absorption wavelength in a wavelength range higher than 400 nm or a precursor for such photosensitizing agent is incorporated in the photosensitizer fluid with a concentration in the range from 0.1 to 2.0 %, and wherein the photosensitizer fluid is delivered to the target area in the form of a number of repeated spray doses during an overall delivery time ranging from 0.25 to 8 hours and the target area is exposed to light energy in a range of wavelengths comprising said at least one absorption wavelength of the photosensitizing agent, the light energy being supplied to the target area in the form of at least one light pulse producing in the target area an accumulated energy density sufficient for photodynamic activation of the photosensitizing agent.

## Patentansprüche

1. Verfahren zur nicht-therapeutischen fotodynamischen Behandlung einer Zielregion der Haut, das die folgenden Schritte umfasst:
Abgabe einer Fotosensibilisatorflüssigkeit, die ein Fotosensibilisierungsmittel oder eine Vorstufe für ein Fotosensibilisierungsmittel beinhaltet, auf die Zielregion, wobei das Fotosensibilisierungsmittel zumindest eine Absorptionswellenlänge in einem Wellenlängenbereich aufweist, der größer ist als 400 nm und
Bestrahlung der Zielregion mit Lichtenergie in einem Wellenlängenbereich, der zumindest eine Absorptionswellenlänge des Fotosensibilisierungsmittels umfasst, wobei die Lichtenergie der Zielregion in Form von zumindest einem Lichtimpuls zugeführt wird, der in der Zielregion eine akkumulierte Energiedichte erzeugt, die für eine fotodynamische Aktivierung des Fotosensibilisierungsmittels ausreichend ist,
**dadurch gekennzeichnet,**
**dass** das Fotosensibilisierungsmittel oder die Vorstufe in die Fotosensibilisatorflüssigkeit mit einer Konzentration im Bereich von 0,1 bis 2,0 % eingebracht wird und dass die Fotosensibilisatorflüssigkeit an die Zielregion in Form einer Anzahl von wiederholten Sprühdosen während einer Gesamtabgabezeit im Bereich von 0,25 bis 8 Stunden abgegeben wird.

2. Verfahren nach Anspruch 1, bei dem die Fotosensibilisatorflüssigkeit ein Fotosensibilisierungsmittel oder eine Vorstufe für ein Fotosensibilisierungsmittel beinhaltet, das/die in der Lage ist, eine Verteilung des Fotosensibilisierungsmittels im Hautgewebe in der Zielregion durch metabolische Transformation zu erzeugen.

3. Verfahren nach Anspruch 2, bei dem die Vorstufe eine Aminolävulinsäure (ALA) oder ein ALA-Ester umfasst, ausgewählt aus ALA-Methylester, ALA-n-pentylester, ALA-n-octylester, R,S-ALA-2-(hydroxymethyl)tetrahydropyranylester, N-acetyl-ALA oder N-acetyl-ALA-ethylester.

4. Verfahren nach einem der Ansprüche 1 bis 3, bei dem die Fotosensibilisatorflüssigkeit ein Liposom-Präparat umfasst, in das das Fotosensibilisierungsmittel oder die Vorstufe durch Einkapselung in Liposombläschen eingebracht ist, wobei das Liposom-Präparat aus einer nanokolloiden Liposom-Emulsion mit einem mittleren Bläschendurchmesser von weniger als 350 nm hergestellt wird.

5. Verfahren nach einem der Ansprüche 1 bis 4, bei dem das Fotosensibilisierungsmittel oder die Vorstufe in die Fotosensibilisatorflüssigkeit mit einer Konzentration im Bereich von 0,1 % bis 0,9 % eingebracht wird.

6. Verfahren nach einem der Ansprüche 1 bis 5, bei dem die Anzahl der wiederholten Sprühdosen zwischen 2 und 36 liegt.

7. Verfahren nach einem der Ansprüche 1 bis 6, bei dem die Sprühdosen an die Zielregion in Intervallen von 2 bis 30 Minuten abgegeben werden.

8. Verfahren nach einem der Ansprüche 1 bis 7, bei dem die Bestrahlung der Zielregion mit Lichtenergie mit einer Zeitverzögerung eingeleitet wird, die 5 Minuten vom Ende der Gesamtabgabezeit für die Fotosensibilisatorflüssigkeit an die Zielregion nicht übersteigt.

9. Verfahren nach einem der Ansprüche 1 bis 8 zur Hautverjüngung, Reduzierung von Falten, Behandlung von Teleangiektasie oder Haarentfernung.

10. Fotosensibilisatorflüssigkeit mit einem Fotosensibilisierungsmittel oder einer Vorstufe von diesem zur Verwendung in einem Verfahren zur therapeutischen fotodynamischen Behandlung einer Zielregion der Haut, das die folgenden Schritte umfasst:
Bereitstellung einer Fotosensibilisatorflüssigkeit mit einem Fotosensibilisierungsmittel mit zumindest einer Absorptionswellenlänge in einem Wellenlängenbereich über 400 nm oder einer Vorstufe für ein derartiges Fotosensibilisierungsmittel mit einer Konzenteration im Bereich von 0,1 bis 2,0 %,
Abgabe der Fotosensibilisatorflüssigkeit an die Zielregion,
Bestrahlung der Zielregion mit Lichtenergie in einem Wellenlängenbereich, der zumindest eine Absorptionswellenlänge des Fotosensibilisierungsmittels umfasst, wobei die Lichtenergie der Zielregion in Form von zumindest einem Lichtimpuls zugeführt wird, der im Zielgebiet eine akkumulierte Energiedichte erzeugt, die für eine fotodynamische Aktivierung des Fotosensibilisierungsmittels ausreichend ist,
**dadurch gekennzeichnet, dass** die Fotosensibilisatorflüssigkeit an die Zielregion in Form einer Anzahl von wiederholten Sprühdosen während einer Gesamtabgabezeit von 0,25 bis 8 Stunden abgegeben wird.

11. Fotosensibilisatorflüssigkeit nach Anspruch 10, zur Verwendung bei einer Behandlung von Akne vulgaris, Narbenakne, Kupferfinnen, Schuppenflechte, Aktinischen Keratosen, Basalzellkarzinomen, Bowen-Krankheit, bösartigen Hautleiden oder Ekzemen.

12. Fotosensibilisatorflüssigkeit nach Anspruch 10 oder 11, wobei die Fotosensibilisatorflüssigkeit ein Liposom-Präparat umfasst, in das das Fotosensibilisierungsmittel oder die Vorstufe durch Einkapselung in Liposombläschen mit einem mittleren Bläschendurchmesser von nicht über 250 nm eingebracht ist.

13. Fotosensibilisatorflüssigkeit nach einem der Ansprüche 10 bis 12, bei der das Fotosensibilisierungsmittel oder die Vorstufe in die Fotosensibilisatorflüssigkeit mit einer Konzentration im Bereich von 0,1 bis 0,9 % eingebracht ist.

14. Verwendung einer Fotosensibilisatorflüssigkeit, die ein Fotosensibilisierungsmittel mit zumindest einer Absorptionswellenlänge in einem Wellenlängenbereich über 400 nm oder eine Vorstufe für ein derartiges Fotosensibilisierungsmittel mit einer Konzentration im Bereich von 0,1 bis 2,0 % zur nicht-therapeutischen fotodynamischen Behandlung einer Zielregion der Haut beinhaltet, wobei die Fotosensibilisatorflüssigkeit an die Zielregion abgegeben wird, die Zielregion mit Lichtenergie in einem Wellenlängenbereich bestrahlt wird, der zumindest eine Absorptionswellenlänge des Fotosensibilisierungsmittels umfasst, die Lichtenergie der Zielregion in Form von zumindest einem Lichtimpuls zugeführt wird, der in der Zielregion eine akkumulierte Energiedichte erzeugt, die zur fotodynamischen Aktivierung des Fotosensibilisierungsmittels ausreichend ist, und die Fotosensibilisatorflüssigkeit an die Zielregion in Form einer Anzahl von wiederholten Sprühdosen während einer Gesamtabgabezeit im Bereich von 0,25 bis 8 Stunden abgegeben wird.

15. Verwendung eines Fotosensibilisierungsmiftels oder einer Vorstufe für ein Fotosensibilisierungsmittel in der Herstellung einer Fotosensibilisatorflüssigkeit zur Anwendung in einer fotodynamischen Behandlung durch Therapie eines Hautleidens, wobei das Fotosensibilisierungsmittel mit zumindest einer Absorptionswellenlänge in einem Wellenlängenbereich von über 400 nm oder eine Vorstufe für ein derartiges Fotosensibilisierungsmittel in die Fotosensibilisatorflüssigkeit mit einer Konzentration im Bereich von 0,1 bis 2 % eingebracht wird, und wobei die Fotosensibilisatorflüssigkeit an die Zielregion in Form einer Anzahl von wiederholten Sprühdosen während einer Gesamtabgabezeit im Bereich von 0,25 bis 8 Stunden abgegeben wird und die Zielregion mit Lichtenergie in einem Wellenlängenbereich bestrahlt wird, der zumindest eine Absorptionswellenlänge des Fotosensibilisierungsmittels umfasst, und die Lichtenergie an die Zielregion in Form von zumindest einem Lichtimpuls abgegeben wird, der in der Zielregion eine akkumulierte Energiedichte erzeugt, die für eine fotodynamische Aktivierung des Fotosensibilisierungsmittels ausreichend ist.

## Revendications

1. Procédé pour le traitement photodynamique non-thérapeutique d'une zone cible de la peau, comprenant les étapes consistant à :
fournir un fluide photosensibilisateur comprenant un agent photosensibilisant ou un précurseur d'un agent photosensibilisant à la zone cible, l'agent photosensibilisant ayant au moins une longueur d'onde d'absorption dans une gamme de longueurs d'onde supérieure à 400 nm, et
exposer ladite zone cible à une énergie lumineuse dans une gamme de longueurs d'onde comprenant ladite au moins une longueur d'onde d'absorption de l'agent photosensibilisant, l'énergie lumineuse étant amenée à la zone cible sous la forme d'au moins une impulsion lumineuse produisant dans une zone cible une densité d'énergie accumulée suffisante pour l'activation photodynamique de l'agent photosensibilisant,
**caractérisé en ce que**
l'agent photosensibilisant ou son précurseur est incorporé dans le fluide photosensibilisateur avec une concentration allant de 0,1 à 2,0 % et que le fluide photosensibilisateur est fourni à la zone cible sous la forme d'un certain nombre de doses de vaporisation répétées pendant une durée de distribution globale allant de 0,25 à 8 heures.

2. Procédé selon la revendication 1, dans lequel le fluide photosensibilisateur comprend un agent photosensibilisant ou un précurseur pour un agent photosensibilisant, capable de produire une distribution de l'agent photosensibilisant dans le tissu dermique dans la zone cible par transformation métabolique.

3. Procédé selon la revendication 2, dans lequel le précurseur comprend un acide aminolévulinique (ALA), ou un ester d'ALA, choisi parmi un méthylester d'ALA, un ALA-n-pentylester, un ALA-n-octylester, un R,S-ALA-2-(hydroxyméthyl)tétrahydropyranyl ester, un N-acétyl-ALA ou un N-acétyl-ALA-éthylester.

4. Procédé selon l'une quelconque des revendications 1 à 3, dans lequel le fluide photosensibilisateur comprend une préparation liposomique comprenant l'agent photosensibilisant ou son précurseur par encapsulation dans des vésicules de liposome, ladite préparation liposomique étant préparée à partir d'une émulsion liposomique nanocolloïde ayant un diamètre de vésicule moyen inférieur à 250 nm.

5. Procédé selon l'une quelconque des revendications 1 à 4, dans lequel l'agent photosensibilisant ou son précurseur est incorporé dans le fluide photosensibilisateur avec une concentration dans la gamme 0,1 à 0,9 %.

6. Procédé selon l'une quelconque des revendications 1 à 5, dans lequel le nombre de doses de vaporisation répétées est entre 2 et 36.

7. Procédé selon l'une quelconque des revendications 1 à 6, dans lequel les doses de vaporisation sont fournies à la zone cible avec des intervalles de 2 à 30 minutes.

8. Procédé selon l'une quelconque des revendications 1 à 7, dans lequel l'exposition de la zone cible à l'énergie lumineuse est débutée, avec un délai ne dépassant pas 5 minutes, à partir de la fin de ladite durée globale de distribution pour le fluide photosensibilisateur à la zone cible.

9. Procédé selon l'une quelconque des revendications 1 à 8 pour un rajeunissement de la peau, la réduction des rides, le traitement de la télangiectasie, ou l'épilation.

10. Fluide photosensibilisant comprenant un agent photosensibilisant ou son précurseur, à utiliser dans un procédé pour le traitement photodynamique thérapeutique d'une zone cible de la peau, comprenant les étapes consistant à fournir un fluide photosensibilisateur comprenant un agent photosensibilisateur ayant au moins une longueur d'onde d'absorption dans une gamme de longueur d'onde supérieure à 400 nm ou un précurseur de cet agent photosensibilisant, avec une concentration dans la gamme de 0,1 à 2,0 %,
fournir le fluide photosensibilisateur à la zone cible,
exposer la zone cible à l'énergie lumineuse dans une gamme de longueurs d'onde comprenant ladite au moins une longueur d'onde d'absorption de l'agent photosensibilisant, l'énergie lumineuse étant fournie à la zone cible sous la forme d'au moins une impulsion lumineuse produisant dans la zone cible une densité d'énergie accumulée suffisante pour une activation photodynamique de l'agent photosensibilisant,
**caractérisé en ce que** le fluide photosensibilisateur est fourni à la zone cible sous la forme d'un certain nombre de doses de vaporisation répétées pendant une durée de distribution globale allant de 0,25 à 8 heures.

11. Fluide photosensibilisant selon la revendication 10, à utiliser dans le traitement de l'acné vulgaire, des cicatrices d'acné, de l'acné rosacée, du psoriasis, de la kératose actinique, du carcinome de cellules basales, de la maladie de Bowen, d'une condition dermatologique maligne ou d'eczéma.

12. Fluide photosensibilisant selon les revendications 10 ou 11, dans lequel le fluide photosensibilisateur comprend une préparation liposomique comprenant l'agent photosensibilisant ou son précurseur par encapsulation dans des vésicules de liposome ayant un diamètre moyen de vésicule ne dépassant pas 250 nm.

13. Fluide photosensibilisant selon l'une quelconque des revendications 10 à 12, dans lequel l'agent photosensibilisant ou son précurseur est incorporé dans le fluide photosensibilisateur avec une concentration dans la gamme de 0,1 à 0,9 %.

14. Utilisation d'un fluide photosensibilisateur incorporant un agent photosensibilisateur, ayant au moins une longueur d'onde d'absorption dans une gamme de longueurs d'onde supérieure à 400 nm ou un précurseur pour cet agent photosensibilisant, avec une concentration dans la gamme de 0,1 à 2,0 % pour un traitement photodynamique non-thérapeutique d'une zone cible de la peau, dans laquelle le fluide photosensibilisateur est fourni à la zone cible, la zone cible est exposée à l'énergie lumineuse dans une gamme de longueurs d'onde comprenant ladite au moins une longueur d'onde d'absorption de l'agent photosensibilisant, l'énergie lumineuse étant fournie à la zone cible sous la forme d'au moins une impulsion lumineuse produisant dans la zone cible une densité d'énergie accumulée suffisante pour une activation photodynamique de l'agent photosensibilisant, et le fluide photosensibilisateur est fourni à la zone cible, sous la forme d'un certain nombre de doses de vaporisation répétées pendant une durée globale de distribution allant de 0,25 à 8 heures.

15. Utilisation d'un agent photosensibilisant ou d'un précurseur pour un agent photosensibilisant dans la fabrication d'un fluide photosensibilisateur, pour application dans le traitement photodynamique par thérapie d'une condition de la peau, dans laquelle l'agent photosensibilisant ayant au moins une longueur d'onde d'absorption dans une gamme de longueurs d'onde supérieure à 400 nm ou un précurseur pour cet agent photosensibilisant est incorporé dans le fluide photosensibilisant avec une concentration dans la gamme de 0,1 à 2,0 % et dans laquelle le fluide photosensibilisant est fourni à la zone cible sous la forme d'un certain nombre de doses de vaporisation répétées, pendant une durée globale de distribution allant de 0,25 à 8 heures, et la zone cible est exposée à l'énergie lumineuse dans une gamme de longueurs d'onde comprenant ladite au moins une longueur d'onde d'absorption de l'agent photosensibilisant, l'énergie lumineuse étant fournie à la zone cible, sous la forme d'au moins une impulsion lumineuse, produisant dans la zone cible une densité d'énergie accumulée suffisante pour une activation photodynamique de l'agent photosensibilisant.
